# EUROPÄISCHE PATENTSCHRIFT

(11) **EP 0 565 826 B1**
(45) Veröffentlichungstag und Bekanntmachung des Hinweises auf die Patenterteilung: **24.04.1996**
(21) Anmeldenummer: 93102186.9
(22) Anmeldetag: 12.02.1993
(51) Int. Cl.: C07C 17/16, C07C 22/00

(54) **Verfahren zur Herstellung von substituiertem oder unsubstituiertem Chlormethylcyclopropan und Brommethylcyclopropan**
Process for the preparation of substituted or unsubstituted chloromethylcyclopropane or bromomethylcyclopropane
Procédé pour la préparation de chlorométhylcyclopropane et bromométhylcyclopropane substitué ou non-substitué

(30) Priorität: 16.04.1992 DE 4212766
(43) Veröffentlichungstag der Anmeldung: 20.10.1993
(73) Patentinhaber: HÜLS AKTIENGESELLSCHAFT, D-45764 Marl (DE)
(72) Erfinder: Kaufhold, Manfred, Dr., W-4370 Marl (DE)

(56) Entgegenhaltungen:
- EP-A- 0 251 246
- HOUBEN-WEYL, "Methoden der Organischen Chemie", 4. Auflage, Band V/4, 1960, Georg Thieme Verlag, Stuttgart, DE, S. 408-411
- HOUBEN-WEYL, "Methoden der Organischen Chemie", 4. Auflage, Band V/3, 1962, Georg Thieme Verlag, Stuttgart, DE, S. 895, 896
- J. ORG. CHEM.,Bd. 49, Nr. 3, 1984, Seiten 431 - 435, R.T. HRUBIEC et al: "Regioselective route to sterically hindered cyclopropylcarbinyl halides", S. 431-435

## Beschreibung

Die Erfindung betrifft ein Verfahren zur Herstellung von Chlor- bzw. Brom-methylcyclopropan der Formel (1) sowie dessen im Ring substituierten Produkte durch Umsetzung von Hydroximethylcyclopropan (2) oder entsprechenden substituierten Verbindungen mit Methansulfonsäurechlorid bzw. - bromid - im folgenden Mesylchlorid bzw. Mesylbromid genannt - in Gegenwart von tertiären Alkylaminen als sogenannte Eintopfreaktion: 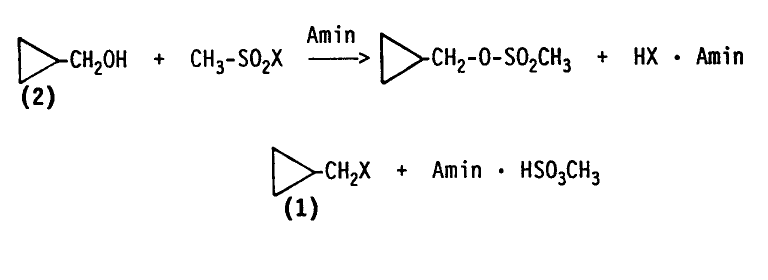
X = Cl, Br
Synthesen von Chloralkanen aus Alkoholen, die wie im Fall 2 zur Isomerisierung neigen, erfordern besondere Maßnahmen. So entsteht z. B. bei der Umsetzung von 2 mit Chlorwasserstoff Chlorcyclobutan-4 als Hauptprodukt in guter Ausbeute: 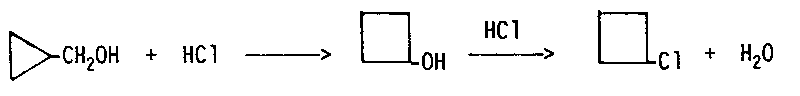
(s. Lee. C. C. und Cessna A. J. Can. J. Chem. 58 (1980) 11, 1075-79)
Um diese Isomerisierung zu unterdrücken, wird in der Literatur das sogenannte Tosylatverfahren empfohlen (s. Weygand/Hilgetag, Organisch-Chemische Experimentierkunst, 4. Auflage, 1970, J. Ambrosius Barth, Leipzig, S. 232). Im Gegensatz zu anderen Verfahren, die intermediär über Sulfinate oder Phosphorigsäureester-Derivate führen, ist das Tosylatverfahren zweistufig. Man stellt zunächst ein Toluolsulfonsäureester (das "Tosylat") oder den Methansulfonsäureester (das "Mesylat") des Alkohols her und setzt diesen in einer zweiten Stufe in einem anderen Lösungsmittel (z. B. 2-Ethoxyethanol oder Aceton) mit einem Alkali- oder Erdalkalimetallhalogenid um.

Die Herstellung der Tosylate und Mesylate erfolgt in bekannter Weise durch Umsetzung der Alkohole mit Tosyl- oder Mesylchlorid in Gegenwart von starken Basen, wie z. B. Pyridin oder Pyridinderivaten (s. obengenannte Literaturstelle und die hier angegebenen Zitate). Die Aufarbeitung erfolgt bei flüssigen Tosylaten und Mesylaten mit Eiswasser-Wäsche, Extraktion und Destillation. Das im großen Überschuß als Lösungsmittel eingesetzte Pyridin wird mit Wasser, eventuell nach vorherigem Ansäuern, vollständig ausgewaschen und wird als wäßrige Lösung verworfen. Eine Aufarbeitung dieses Abwassers zur Rückgewinnung des wasserlöslichen Pyridins ist sehr aufwendig.
Bei der zweiten Stufe der Umsetzung der Tosylate oder Mesylate mit einem Alkali- oder Erdalkalihalogenid wird ebenfalls ein polares wasserlösliches Lösungsmittel benötigt. Seine Abtrennung vom entstehenden Alkali- oder Erdalkalisalz der Toluol- oder Methansulfonsäure ist auch nur mit aufwendigen technischen Maßnahmen möglich.

Alle bekannten Tosylat- und Mesylat-Verfahren haben einen hohen Verbrauch an Chemikalien, sind technisch aufwendig und zeitraubend und führen zu Problemen in der Abfallbeseitigung. Wünschenswert ist daher ein Verfahren, bei dem z. B. Hydroximethylcyclopropan mit Mesylchlorid bzw. -bromid in das Chlor- bzw. Brom-methylcyclopropan überführt werden kann und die dabei benutzten Lösungsmittel leicht zurückgewonnen werden können. Es besteht ein großes Interesse an einem derartigen Verfahren, nach dem man bei geringem technischen Aufwand und ohne Verbrauch von umweltschädlichen Reagenzien wie Pyridin und Alkylglykolether aus z. B. Hydroximethylcyclopropan Chlor- und/oder Brom-methylcyclopropan herstellen kann, und zwar mit einer Reinheit von über 85 %, vorzugsweise über 90 %, weil diese Produkte wichtige Rohstoffe für Pharma-Produkte sind.

Die sich hieraus ergebende Aufgabe wird dadurch gelöst, daß überraschenderweise gefunden wurde, daß z. B. bei der Umsetzung des Hydroximethylcyclopropans mit Mesylhalogenid das wasserlösliche Pyridin vorteilhaft durch wenig- oder sogar wasserunlösliche Trialkylamine ersetzt werden kann. Ferner wurde überraschenderweise gefunden, daß bei geeigneter Temperaturführung in Kombination mit genau gewählter Zeit der Zugabe des Mesylhalogenids bei dieser Umsetzung die Chlor- bzw. Brom-methylcyclopropane direkt in guter Ausbeute entstehen, d. h. das entstehende Mesylat nicht isoliert werden muß, sondern gleich weiter zum Zielprodukt reagiert.

Gegenstand der Erfindung ist ein Verfahren zur Herstellung von substituiertem oder unsubstituiertem Chlor- bzw. Brommethylcyclopropan mit Reinheitsgraden über 85 %, insbesondere über 90 %, durch Umsetzung von Hydroximethylcyclopropan der allgemeinen Formel 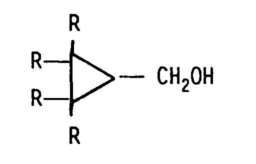
worin R = H oder 1 bis 2 R auch entweder Alkyl mit 1 bis 10 C-Atomen oder -Phenyl bedeuten können,
mit Methansulfonsäurechlorid bzw. -bromid, welches dadurch gekennzeichnet ist, daß die Umsetzung in Gegenwart von Trialkylaminen und gegebenenfalls O-haltigen organischen Lösungsmitteln ohne Isolierung des Mesylats erfolgt, wobei man Hydroximethylcyclopropan und Trialkylamin im Molverhältnis 1 : 1 bis 1 : 10 einsetzt und das Methylsulfonsäurechlorid bzw. -bromid entweder innerhalb von 1 bis 3 Stunden bei -5 °C bis -20 °C, oder innerhalb von 3 bis 10 Stunden bei -5 °C bis +5 °C, oder innerhalb von 10 bis 20 Stunden bei +5 °C bis 15 °C, oder in 20 bis 30 Stunden bei +20 °C bis +30 °C, zugibt, um ein reines, d. h. 85 bis 90 %iges Chlor- bzw. Brom-methylcyclopropan, zu erhalten, und nach dieser Zugabe das Produktgemisch auf +40 bis +80 °C bzw. auf Rückflußtemperatur erwärmt.

Diese erfindungsgemäße Lösung steht im Gegensatz zu der obengenannten Literaturstelle, die besagt, daß bei der Umsetzung von Alkoholen mit Tosyl- und Mesylchlorid zwar neben anderen Verbindungen auch Halogenverbindungen entstehen können, daß man aber zur Herstellung von reinen Halogenverbindungen zunächst die Tosylate oder Mesylate herstellen muß und diese in einer zweiten Stufe in sauerstoffhaltigen polaren Lösungsmitteln mit einem Alkali- oder Erdalkalihalogenid umsetzt.

Ein Vorteil dieses erfindungsgemäßen Verfahrens ist also u. a. die leichte Rückführung des Lösungsmittels, das überraschender Weise für beide Stufen gut einsetzbar ist.

Geeignete Lösungsmittel sind z. B. Ether, wie Diethylether, Dipropylether, Diisopropylether und besonders Methyl-tert.-butylether. Die Lösungsmittelmenge beträgt das 2- bis 10fache der Menge an eingesetztem Alkohol, vorzugsweise das 1,1- bis 1,3fache.

Die Trialkylamine können symmetrisch oder unsymmetrisch sein und allein oder im Gemisch verwendet werden. Bevorzugte Trialkylamine sind aus wirtschaftlichen Gründen die üblichen marktgängigen, kurzkettigen Amine, wie Triethyl-, Tripropyl-, Tributyl-, Tripentyl-, Trihexyl- Triheptyl- und Trioctylamin. Wegen seines günstigen Siedepunktes und seiner geringen Wasserlöslichkeit wird Tributylamin bevorzugt eingesetzt. Die unsymmetrischen Trialkylamine, die eingesetzt werden können, entsprechen der allgemeinen Formel 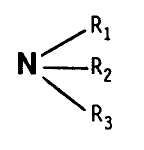
worin R₁ und R₂ = CH₃, -C₂H₅
R₃ = C₃H₇
und im Falle von R₁ = R₂ = CH₃ oder C₂H₅ kann R₃ auch -C₄H₉, -C₅H₁₁, -C₆H₁₃, -C₇H₁₅, -C₈H₁₇, -C₁₀H₂₁, -C₁₂H₂₅ bedeuten.

Die praktische Durchführung erfolgt beispielsweise in folgender Weise: Hydroximethylcyclopropan (HMCP), ein Lösungsmittel, z. B. Methyl-tert.-butylether und Tributylamin, werden vorgelegt, wobei das Molverhältnis HMCP zum Amin vorzugsweise 1 : 1 bis 1 : 2, und besonders bevorzugt 1 : 1,1 bis 1 : 1,3, beträgt. Die Gewichtsmenge an Lösungsmittel beträgt das 2- bis 10fache, bevorzugt das 1,1- bis 1,3fache, der Gewichtsmenge an HMCP. Die Temperatur des Reaktionsgemisches wird auf -20 °C bis + 30 °C, bevorzugt auf -5 °C bis 0 °C, eingestellt. Innerhalb von 3 bis 30 Stunden wird Mesylhalogenid zugetropft und die Temperatur durch Kühlung gehalten. Das Molverhältnis HMCP zu Mesylhalogenid beträgt 1 : 1 bis 1 : 2, vorzugsweise 1 : 1,01 bis 1 : 1,1. Das Amin kann vorgelegt oder auch gleichzeitig mit dem Mesylchlorid zum Reaktionsgemisch zudosiert werden.

Die Temperatur des Reaktionsgemisches wird auf -20 °C bis +30 °C, bevorzugt auf -10 °C bis +20 °C, insbesondere auf -5 °C bis 0 °C, eingestellt.

Die Zutropfzeit für das Mesylhalogenid ist abhängig von der gewählten Reaktionstemperatur, wenn man das Ziel der Erfindung, reines 85 bis 90 %iges Chlor- bzw. Brom-methylcyclopropan herstellen will: Je tiefer die Temperatur gewählt wird, um so schneller kann das Mesylhalogenid zugegeben werden. Bei -10 °C führt eine Zutropfzeit von 3 h zu einem Reinheitsgrad von ca. 93 %, während bei 25 °C die Zutropfzeit auf 23 Stunden verlängert werden muß, um zu diesem Reinheitsgrad zu gelangen.

Nach der Zugabe des Mesylhalogenids wird das Reaktionsgemisch durch Entfernen der Kühlung innerhalb von 1 bis 2 Stunden auf Raumtemperatur gebracht, wenn eine tiefere Reaktionstemperatur gewählt wurde. Dann wird innerhalb von 1 bis 2 Stunden die Temperatur langsam erhöht, bis sich Rückfluß einstellt. Zwei Stunden wird am Rückfluß zum Sieden erhitzt und anschließend auf 0 bis 10 °C abgekühlt.

Anschließend wird das Reaktionsgemisch mit basischen Mitteln, wie Natronlauge, Kalilauge, Calciumhydroxid und anderen Hydroxiden oder mit Alkoholaten, wie Natriummethylat, Kaliummethylat, Natriumethylat usw., alkalisch eingestellt. Das basische Mittel kann als Feststoff oder als Lösung eingesetzt werden.

Setzt man eine wäßrige Lösung eines Metallhydroxids zur Neutralisation ein, trennt sich das Gemisch in zwei Phasen, von denen die wäßrige verworfen und die Ölphase destillativ aufgearbeitet wird.

Diese Destillation liefert das gewünschte Chlor- bzw. Brom-methylcyclopropan in einer Ausbeute von 75 bis 80 % und einer Reinheit von über 90 % und als höhersiedende Fraktion das eingesetzte Trialkylamin zurück.

Den folgenden Beispielen ist weiteres Zahlenmaterial zu entnehmen; sie sollen das erfindungsgemäße Verfahren näher erläutern.

### Beispiel 1

Man benutzt eine Glasapparatur, die aus eines Dreihalskolben mit Rührer, Thermometer, Tropftrichter und Rückflußkühler besteht.
Man setzt ein:
- 144 g (1,97 Mol): Hydroximethylcyclopropan (98,6 %ig)
- 411 g (2,2 Mol): Tributylamin (99 %ig)
- 300 g: Methyl-tert.-butylether
als Lösungsmittel
Diese Einsatzprodukte werden vorgelegt und unter Rühren auf -10 °C gekühlt. Dann wird innerhalb von 3 Stunden 232 g (2,0 Mol) Mesylchlorid (99 %ig) unter starker Kühlung bei -10 °C bis -5 °C zugetropft. Anschließend wird die Temperatur innerhalb von einer Stunde auf -5 °C auf +3 °C erhöht. Innerhalb einer weiteren Stunden wird auf 30 °C und danach in einer halben Stunde auf Rückflußtemperatur, die bei 64 °C liegt, erwärmt. Zwei Stunden wird am Rückfluß zum Sieden erhitzt und anschließend auf 10 °C abgekühlt.
Zur Neutralisation des Methansulfonsäuresalzes des Tributylamins wird bei 10 °C 430 g (2,15 Mol) Natronlauge (20 %ig) zum Reaktionsgemisch gegeben und danach die Phasen getrennt. Die wäßrige Phase, 610 g, wird verworfen und die Ölphase, 865 g, wird destillativ aufgearbeitet. Man erhält in einem Siedebereich von 83 bis 93 °C bei Normaldruck 154 g Chlormethylcyclopropan mit einer Reinheit von 93 %. Die Ausbeute beträgt 80,5 % der Theorie, bezogen auf Einsatz.
In einem Siedebereich von 100 bis 104 °C bei 33 mbar fallen 388 g Tributylamin mit einer Reinheit von 99,2 %, das direkt wieder eingesetzt werden kann. Aus dem Destillationsrückstand, 24 g, erhält man nach Neutralisation mit 25 %iger Natronlauge im Überschuß weitere 10 g Tributylamin.

### Beispiel 2

Man benutzt die im Beispiel 1 beschriebene Apparatur, setzt die dort genannten Produkte ein, kühlt das Gemisch aber nicht ab, sondern stellt eine Temperatur von 20 °C ein und tropft das Mesylchlorid in 9 Stunden zu. Bei gleicher Weiterverarbeitung wie im Beispiel 1 erhält man ein Chlormethylcyclopropan mit 89 %iger Reinheit in 79 %iger Ausbeute der Theorie, bezogen auf Einsatz.

### Beispiele 3 und 4

Man benutzt die im Beispiel 1 beschriebene Apparatur, setzt die dort genannten Produkte ein und stellt, wie im Beispiel 2, eine Temperatur von 20 °C ein. Die Zutropfzeit beträgt beim Beispiel 3 2 Stunden und beim Beispiel 4 23 Stunden. Bei gleicher Weiterverarbeitung wie in den Beispielen 1 und 2 wird Chlormethylcyclopropan mit 73 %iger bzw. 93 %iger Reinheit erhalten. Die Ausbeuten liegen bei ca. 78 bzw. 81 % der Theorie, bezogen auf Einsatz.

### Beispiel 5

Man benutzt die im Beispiel 1 beschriebene Apparatur.
Man setzt ein:
- 65 g (0,9 Mol): Hydroximethylcyclopropan (98,6 %ig)
- 216 g (1,15 Mol): Tributylamin (99 %ig)
- 200 g: Methyl-tert.-butylether
als Lösungsmittel
Diese Einsatzprodukte werden vorgelegt und unter Rühren auf -10 °C abgekühlt. Dann wird innerhalb von 2,5 Stunden bei -10 °C bis -5 °C 169 g (0,9 Mol) Mesylbromid (85 %ig) zugetropft. Anschließend wird eine Stunde bei -5 °C gerührt und dann innerhalb von einer Stunde bis zum Sieden am Rückfluß erwärmt. Nach halbstündigem Sieden am Rückfluß - die Temperatur liegt bei ca. 64 °C - wird auf 10 °C abgekühlt und mit 215 g 20 %iger Natronlauge das Gemisch alkalisch eingestellt.

Die Phasentrennung ergibt:

| | |
|---|---|
| Ölphasse | 531 g |
| wäßrige Phase | 325 g |

Die gaschromatografische Analyse zeigt neben einer Reihe nicht bekannter Verbindungen folgende Gehalte an:

| | |
|---|---|
| Methyl-tert.-butylether | 36,1 % |
| Brommethylcyclopropan | 15,6 % |
| Tributylamin | 45,0 % |

Hieraus errechnet sich eine Ausbeute der Theorie, bezogen auf Einsatz, von ca. 68 %.
Die Reinheit des Brommethylcyclopropans nach Aufarbeitung beträgt 89,0 %.

### Beispiel 6

Man benutzt die im Beispiel 1 beschriebene Apparatur und setzt die dort genannten Produkte ein, aber in diesem Fall an Stelle von 2,2 Mol Tributylamin setzt man 2,2 Mol Methyl-, Butyl-, Octylamin ein. Nach weiterer Durchführung wie im Beispiel 1 erhält man Chlormethylcyclopropan mit 91 %iger Reinheit in einer Ausbeute von 81 % der Theorie.

### Beispiel 7

Man arbeitet wie im Beispiel 6 beschrieben, setzt aber in diesem Fall an Stelle des Methyl-, Butyl-, Octylamins nun 2,2 Mol Dimethyl-dodecylamin ein. Nach analoger Weiterarbeit erhält man Chlormethylcyclopropan mit 93 %iger Reinheit in einer Ausbeute von 78 % der Theorie.

### Beispiel 8

Man benutzt die im Beispiel 1 beschriebene Apparatur und setzt die dort genannten Produkte ein, aber in diesem Fall an Stelle von Hydroximethylcyclopropan nun 2,2-Dimethyl-hydroximethylcyclopropan. Nach weiterer Durchführung wie im Beispiel 1 erhält man 2,2-Dimethyl-chlormethylcyclopropan mit 89 %iger Reinheit in einer Ausbeute von 81 % der Theorie.

## Patentansprüche

1. Verfahren zur Herstellung von substituierten oder unsubstituierten Chlor- bzw. Brom-methylcyclopropan mit Reinheitsgraden über 85 %, insbesondere über 90 %, in Lösungsmitteln durch Umsetzung von Hydroximethylcyclopropan der allgemeinen Formel 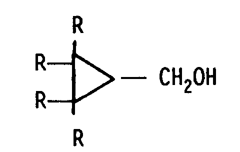 worin R = H oder 1 bis 2 R auch entweder Alkyl mit 1 bis 10 C-Atomen oder -Phenyl bedeuten können,
mit Methansulfonsäurechlorid bzw. -bromid,
dadurch gekennzeichnet,
daß die Umsetzung in Gegenwart von Trialkylaminen und gegebenenfalls O-haltigen organischen Lösungsmitteln ohne Isolierung des Mesylats erfolgt, wobei man Hydroximethylcyclopropan und Trialkylamin im Molverhältnis 1 : 1 bis 1 : 10 einsetzt und das Methansulfonsäurechlorid bzw. -bromid entweder innerhalb von 1 bis 3 Stunden bei -5 °C bis -20 °C, oder innerhalb von 3 bis 10 Stunden bei -5 °C bis +5 °C, oder innerhalb von 10 bis 20 Stunden bei +5 °C bis 15 °C, oder in 20 bis 30 Stunden bei +20 °C bis +30 °C, zugibt und nach dieser Zugabe das Produktgemisch auf 40 bis 80 °C bzw. auf Rückflußtemperatur erwärmt.

2. Verfahren nach Anspruch 1,
dadurch gekennzeichnet,
daß man symmetrische oder unsymmetrische Trialkylamine einsetzt.

3. Verfahren nach den Ansprüchen 1 und 2,
dadurch gekennzeichnet,
daß man als Trialkylamin Triethyl-, -propyl oder n- oder Isobutylamin einsetzt.

4. Verfahren nach Anspruch 1,
dadurch gekennzeichnet,
daß man Hydroximethylcyclopropan zum Amin im Molverhältnis 1 : 1 bis 1 : 2, und bevorzugt 1 : 1,1 bis 1 : 1,3, einsetzt.

5. Verfahren nach Anspruch 1,
dadurch gekennzeichnet,
daß man HMCP zu Mesylhalogenid im Molverhältnis 1 : 1 bis 1 : 2, bevorzugt 1 : 1,01 bis 1 : 1,1, einsetzt.

6. Verfahren nach Anspruch 1,
dadurch gekennzeichnet,
daß das Lösungsmittel ein Ether ist und die 2- bis 10fache Gewichtsmenge, bezogen auf eingesetztes HMCP, beträgt.

7. Verfahren nach den Ansprüchen 1 und 6,
dadurch gekennzeichnet,
daß das Lösungsmittel Diethylether, Dipropylether und Methyl-tert.-butylether ist.

## Claims

1. A process for the preparation of substituted or unsubstituted chloro- or bromo-methylcyclopropane with degrees of purity of over 85 %, in particular over 90 %, in solvents by reacting hydroxymethylcyclopropane of the general formula 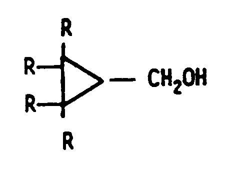 in which R = H or, alternatively, 1 to 2 radicals R may either be alkyl of 1 to 10 carbon atoms or -phenyl, with methanesulphonyl chloride or bromide, respectively,
characterized in that the reaction takes place in the presence of trialkylamine and, if desired, O-containing organic solvent without isolation of the mesylate, with hydroxymethylcyclopropane and trialkylamine being employed in a molar ratio of from 1 : 1 to 1 : 10 and the methanesulphonyl chloride or bromide being added alternatively over the course of from 1 to 3 hours at from -5°C to -20°C or over the course of from 3 to 10 hours at from -5°C to +5°C or over the course of from 10 to 20 hours at from +5°C to 15°C or over from 20 to 30 hours at from +20°C to +30°C, and the product mixture, after this addition, being heated to from 40 to 80°C or to reflux temperature.

2. A process according to claim 1, characterized in that a symmetrical or asymmetrical trialkylamine is employed.

3. A process according to either of claims 1 and 2, characterized in that triethylamine, tripropylamine, tri-n-butylamine or triisobutylamine is employed as trialkylamine.

4. A process according to claim 1, characterized in that the molar ratio employed of hydroxymethylcyclopropane to the amine is from 1 : 1 to 1 : 2, and preferably from 1 : 1.1 to 1 : 1.3.

5. A process according to claim 1, characterized in that the molar ratio employed of HMCP to mesyl halide is from 1 : 1 to 1 : 2, preferably from 1 : 1.01 to 1 : 1.1.

6. A process according to claim 1, characterized in that the solvent is an ether and amounts to from 2 to 10 times the amount by weight of HMCP employed.

7. A process according to either of claims 1 and 6, characterized in that the solvent is diethyl ether, dipropyl ether or methyl tert-butyl ether.

## Revendications

1. Procédé pour préparer du chloro- ou du bromométhylcyclopropane substitué ou non substitué, ayant un degré de pureté supérieur à 85 %, notamment supérieur à 90 %, dans des solvants, par réaction de l'hydroxyméthylcyclopropane, de formule générale 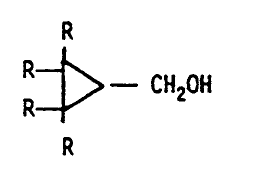 dans laquelle R est H, ou encore 1 ou 2 radicaux R peuvent aussi être des groupes alkyle ayant de 1 à 10 atomes de carbone ou phényle, avec du chlorure ou du bromure de méthanesulfonyle,
caractérisé en ce que la réaction a lieu en présence de trialkylamines et éventuellement de solvants organiques oxygénés, sans isolement du mésylate, auquel cas on utilise l'hydroxyméthylcyclopropane et la trialkylamine selon un rapport en moles de 1 : 1 à 1 : 10, et on ajoute le chlorure ou le bromure de méthanesulfonyle soit en 1 à 3 heures à une température de - 5 à - 20°C, soit en 3 à 10 heures à une température de - 5 à + 5°C, soit en 10 à 20 heures à une température de + 5 à 15°C, soit en 20 à 30 heures à une température de + 20 à + 30°C, et, après cette addition, on chauffe le mélange produit à une température de 40 à 80°C ou à la température de reflux.

2. Procédé selon la revendication 1,
caractérisé en ce qu'on utilise des trialkylamines symétriques ou asymétriques.

3. Procédé selon les revendications 1 et 2,
caractérisé en ce qu'on utilise comme trialkylamine la triéthylamine, la tripropylamine, la tri-n-butylamine ou la triisobutylamine.

4. Procédé selon la revendication 1,
caractérisé en ce qu'on utilise l'hydroxyméthylcyclopropane selon un rapport en moles, par rapport à l'amine, de 1 : 1 à 1 : 2 et de préférence de 1 : 1,1 à 1 : 1,3.

5. Procédé selon la revendication 1,
caractérisé en ce qu'on utilise le HMCP selon un rapport en moles, par rapport à l'halogénure de mésyle, de 1 : 1 à 1 : 2 et de préférence de 1 : 1,01 à 1 : 1,1.

6. Procédé selon la revendication 1,
caractérisé en ce que le solvant est un éther, et que sa quantité est de 2 à 10 fois supérieure à la quantité utilisée du HMCP.

7. Procédé selon les revendications 1 à 6,
caractérisé en ce que le solvant est l'oxyde de diéthyle, l'oxyde de dipropyle et l'oxyde de méthyle et de tert-butyle.
